# EUROPEAN PATENT SPECIFICATION

(11) **EP 0 464 022 B1**
(45) Date of publication and mention of the grant of the patent: **31.05.2000**
(21) Application number: 89910254.5
(22) Date of filing: 20.03.1989
(51) Int. Cl.: C07K 14/605, C07K 14/62, A61K 38/26, A61K 38/28

(54) **INSULINOTROPIC HORMONE**
INSULINOTROPES HORMON
HORMONE INSULINOTROPE

(43) Date of publication of application: 08.01.1992
(73) Proprietor: THE GENERAL HOSPITAL CORPORATION, Boston, MA 02114 (US)
(72) Inventor: HABENER, Joel, F., Newton Highlands, MA 02161 (US)
(74) Representative: Sheard, Andrew Gregory
(86) International application number: US8901121
(87) International publication number: WO9011296

(56) References cited:
- EP-A- 0 044 168
- WO-A-87/06941
- THE LANCET, 5th December 1987, pages 1300-1303; B. KREYMANN et al.: "Glucagon-like peptide-1 7-36: A physiological incretin in man"
- GLUCAGON AND RELATED PEPTIDES PROCEEDINGS OF INTERNATIONAL SYMPOSIUM, Osaka, 15th - 16th July 1988, Editors S. TARUI et al.; & BIOMEDICAL RESEARCH, vol. 9, supplement 3, 1988, pages 201-206; "R. KOMATSU et al.: "Effect of glucagon-related peptides on rat endocrine pancreas"
- DIABETOLOGIA, Volume 27, issued 1984, (GHIGLIONE et al.), "How GLUCAGON-Like is GLUCAGON-Like, peptide-1?", Pages 599-600, See page 600 in particular.
- NATURE, Volume 302, issued 21 April 1983, (BELL et al.), "Hamster Preproglucagon contains the sequence of Glucagon and two related peptides", pages 716-718, See Fig. 1 in particular.
- DIABETOLOGIA, Volume 28, issued 1985, (SCHMIDT et al.), "Glucagon-Like peptide-1 but not Glucagon-Like peptide-2 stimulates insulin release from isolated rat pancreatic islets", pages 704-707, See the summary and pages 706, 707 in particular.
- THE JOURNAL OF CLINICAL ENDOCRINOLOGY AND METABOLISM, Volume 61, issued 1985, (UTTENTHAL et al.), "Molecular forms of Glucagon-like peptide-1 in human pancreas and Glucagonomas", pages 472-479. See the Abstract in particular.
- THE JOURNAL OF BIOLOGICAL CHEMISTRY, Volume 260, issued 10 April 1985, (ANDREWS et al.), "Isolation and structures of Glucagon and Glucagon-like peptide from catfish pancreas", pages 3910-3914. See Fig. 7 in particular.

## Description

### BACKGROUND OF THE INVENTION

### Field of the Invention

This invention is directed to the discovery that certain peptide fragments of the prehormone, proglucagon, possess hormonal activities and can be used to stimulate the synthesis and secretion of the hormone, insulin. These peptide fragments are useful in therapy for the disease Diabetes mellitus.

### Description of the Background Art

The endocrine secretions of the pancreatic islets are under complex control not only by blood-borne metabolites (glucose, amino acids, catecholamines, etc.), but also by local paracrine influences. The major pancreatic islet hormones (glucagon, insulin, and somatostatin) interact among their specific cell types (A, B, and D cells, respectively) to modulate secretory responses mediated by the metabolites. Although insulin secretion is predominantly controlled by blood levels of glucose, glucagon and somatostatin stimulate and inhibit glucose-mediated insulin secretory responses, respectively. In addition to the proposed interislet paracrine regulation of insulin secretion, there is evidence to support the existence of insulinotropic factors in the intestine. This concept originates from the observations that glucose taken orally is a much more potent stimulant of insulin secretion than is a comparable amount of glucose given intravenously.

The human hormone, glucagon, is a 29-amino acid peptide hormone produced in the A-cells of the pancreas. The hormone belongs to a multi-gene family of structurally related peptides that include secretin, gastric inhibitory peptide, vasoactive intestinal peptide, and glicentin. These peptides variously regulate carbohydrate metabolism, gastrointestinal mobility, and secretory processing. The principal recognized actions of pancreatic glucagon, however, are to promote glycogenolysis and gluconeogenesis, resulting in an elevation of blood sugar levels. In this regard, the actions of glucagon are counter-regulatory to those of insulin and may contribute to the hyperglycemia that accompanies Diabetes mellitus (Lund, P.K., et al., Proc. Natl. Acad. Sci., USA 79:345-349 (1982)).

Glucagon has been found to be capable of binding to specific receptors which lie on the surface of insulin-producing cells. Glucagon, when bound to these receptors, stimulates the rapid synthesis of cAMP, by these cells. cAMP, in turn, has been found to stimulate insulin expression (Korman, L.Y., et al., Diabetes 34:717-722 (1985)). Insulin acts to inhibit glucagon synthesis (Review of Medical Physiology, Ganong, W.F., 1979, Lang Publications, Los Altos, California (p. 273)). Thus, the expression of glucagon is carefully regulated by insulin, and ultimately by the serum glucose level.

The glucagon gene is initially translated from a 630-base pair precursor to form the polypeptide, preproglycagon (Lund et al. (1982)). This polypeptide is subsequently processed to form proglucagon. Patzelt, C., et al. (Nature 282:260-266 (1979)) demonstrated that proglucagon was subsequently cleaved into glucagon and a second polypeptide. Subsequent work by Lund, P.K., et al. (Proc. Natl. Acad. Sci. USA 79:345-349 (1982)); Lopez, L.C., et al. (Proc. Natl. Acad. Sci. USA 80:5485-5489 (1983)) and Bell, G.I., et al. (Nature 302:716-718 (1983)) demonstrated that the proglucagon molecule was cleaved immediately after lysine-arginine dipeptide residues. Studies of proglucagon produced by channel catfish (Ictalurus punctata) indicated that glucagon from this animal was also proteolytically cleaved after adjacent lysine-arginine and arginine-arginine dipeptide residues (Andrews, P.C., et al., J. Biol. Chem. 260:3910-3914 (1985)). Lopez, L.C., et al. (Proc. Natl. Acad. Sci. USA 80:5485-5489 (1983)), and Bell, G.I., et al., discovered the mammalian proglucagon was cleaved at lysine-arginine or arginine-arginine dipeptides and demonstrated that the proglucagon molecule contained three discrete and highly homologous peptide molecules which were designated glucagon, glucagon-like protein 1 (GLP-1), and glucagon-like protein 2 (GLP-2). Lopez et al. concluded that glucagon-like protein 1 was 37 amino acid residues long and that glucagon-like peptide 2 was 34 amino acid residues long. Analogous studies on the structure of rat preproglucagon revealed a similar pattern of proteolytic cleavage between adjacent lysine-arginine or arginine-arginine dipeptide residues, resulting in the formation of glucagon, GLP-1, and GLP-2 (Heinrich, G., et al., Endocrinol. 115:2176-2181 (1984)). Human rat, bovine, and hamster sequences of GLP-1 have been found to be identical (Ghiglione, M., et al., Diabetologia 27:599-600 (1984)).

The conclusion reached by topez et al. (Proc. Natl. Acad. Sci. USA 80:5485-5489 (1983)) regarding the size of GLP-1 was confirmed by the work of Uttenthal, L.O., et al., (J. Clin. Endocrinol. Metabol. 61:472-479 (1985)). Uttenthal et al. examined the molecular forms of GLP-1 which were present in the human pancreas. Their research shows that GLP-1 and GLP-2 are present in the pancreas as proteins having 37 and 34 amino acid residues, respectively.

The similarity between GLP-1 and glucagon suggested to early investigators that GLP-1 might have biological activity. Although some investigators found that GLP-1 could induce rat brain cells to synthesize cAMP (Hoosein, N.M., et al., FEBS Lett. 178:83-86 (1984)), other investigators failed to identify any physiological role for GLP-1 (Lopez, L.C, et al., Proc. Natl. Acad. Sci. USA 80:5485-5489 (1983)). The failure to identify any physiological role for GLP-1 caused some investigators to question whether GLP-1 was in fact a hormone and whether the relatedness between glucagon and GLP-1 might be artifactual (Ghiglione, M., et al., Diabetologia 27:599-600 (1984)).

Thus, in conclusion, the prior art reveals an awareness of the processing of a glucagon hormone precursor into a set of peptides sharing extensive homology. It has been widely assumed by those of skill in the art that these highly related glucagon-like peptides would have a biological activity. Nevertheless, extensive investigations designed to elucidate the biological effects of these molecules had been unsuccessful.

### SUMMARY OF THE INVENTION

The present invention relates to a compound which is:
(A) a peptide having the sequence:
   His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys; or
   His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly; or
(B) a peptide having at least 80% homology with one or other of the peptides of (A) with the proviso that the peptide is not GLP-1 7-36, GLP-1 7-37 or GLP-1 1-36; or
(C) a peptide which is:
   (i) a pharmaceutically acceptable acid addition salt of the peptides of (A) or (B);
   (ii) a pharmaceutically acceptable carboxylate salt of the peptides of (A) or (B);
   (iii) a pharmaceutically acceptable lower alkyl ester of the (A) or (B) peptides; and/or
   (iv) a pharmaceutically acceptable amide, alkyl amide, or dialkyl amide of (i), (ii), and/or (iii);
wherein the compound is substantially free of natural contaminants, and has an insulinotropic activity which exceeds the insulinotropic activity of GLP-1 (1-36) or GLP-1 (1-37).

In particular, the invention relates to a compound of the invention which is a peptide having the sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys; or
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly.

Preferably, the compounds of the invention are substantially free of natural contaminants, and have an insulinotropic activity at a concentration of at least 10⁻¹¹M, and most preferably at a concentration of at least 10⁻¹⁰M.

In another aspect the invention also provides a compound having the general formula:
(1)

   H₂N- - X - - CO-R¹

   wherein
   R¹ represents OH, OM or -NR²R³;
   M is a pharmaceutically acceptable cation or a lower branched or unbranched alkyl group;
   R² and R³ are the same or different and each individually represents a hydrogen atom or a lower branched or unbranced alkyl group;
   X is a peptide comprising the sequence:
      His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys; or
      His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
   where NH₂ is the amine group of the amino terminus of X, and with the proviso that X is not GLP-1 7-36, GLP-1 7-37, GLP-1 1-36 or GLP-1 1-37. ; and
   CO is the carbonyl group of the carboxy terminus of X; or
(2) an acid addition salt thereof;
(3) the protected or partially protected derivatives thereof;
wherein the compound has an insulinotropic activity which exceeds the insulinotropic activity of GLP-1 (1-36) or GLP-1 (1-37).

In further aspects the invention provides
(i) an insulinotropic medicament comprising an effective amount of a compound of the invention in combination with a suitable physiologically acceptable carrier;
(ii) a compound of the invention for use in medicine; and
(iii) the use of the compound of the invention in the preparation of an agent for enhancing the expression of insulin in a mammalian pancreatic B-type islet cell.

The prior art documents WO-A-8706941, *The Lancet*, 5 December 1987, 1300-1303 and *Glucagon and Related Peptides Proceedings of International Symposium*, Osaka, 15-16 July 1988 and *Biomedical Research*, **9(3)**, 201-206 (1988) all relate to glucagon-like peptides but these documents disclose GLP-1(7-36) and (7-37).

### BRIEF DESCRIPTION OF THE DRAWINGS

Figure 1 shows the DNA structure and corresponding amino acid sequence of rat preproglucagon. The preproglucagon precursor is proteolytically cleaved at sites indicated by circles.
Figure 2 shows the ability of the insulinotropic peptides glucagon and GLP-1 (7-37) to stimulate cAMP formation in the insulinoma line RIN 1046-38.
Figure 3 shows a comparison of the insulinotropic activity of glucagon with that of GLP-1 (7-37).
Figure 4 shows a comparison of the insulinotropic activities of GLP-1 (7-34), GLP-1 (7-35) and GLP-1 (7-37) using the rat pancreas perfusion technique.
Figure 5 shows the breakdown of GLP-1 (1-37) into GLP-1 (7-37) under experimental conditions.

### DESCRIPTION OF THE PREFERRED EMBODIMENTS

### A. GLP-1 and Its Derivatives

The hormone glucagon is known to be synthesized as a high molecular weight precursor molecule which is subsequently proteolytically cleaved into three peptides: glucagon, glucagon-like peptide 1 (GLP-1), and glucagon-like peptide 2 (GLP-2). GLP-1 has 37 amino acids in its unprocessed form. The present invention discloses that the unprocessed GLP-1 is essentially unable to mediate the induction of insulin biosynthesis. The unprocessed GLP-1 peptide is, however, naturally converted to a 31-amino acid long peptide (7-37 peptide) having amino acids 7-37 of GLP-1 ("GLP-1 (7-37)"). This processing occurs in the pancreas and the intestine. The 7-37 peptide which has not been previosly described is a hormone that has insulinotropic activity. A compound is said to have an "insulinotropic activity" if it is able to stimulate, or cause the stimulation of, the synthesis or expression of the hormone insulin. The hormonal activity of GLP-1 (7-37) appears to be specific for the pancreatic beta cells where it appears to induce the biosynthesis of insulin. The insulinotropic hormone is useful in the study of the pathogenesis of maturity onset diabetes mellitus, a condition in which the dynamics of insulin secretion are abnormal. Moreover, the insulinotropic hormone is useful in therapy for this disease.

Peptide moieties (fragments) chosen from the determined amino acid sequence of human GLP-1 constitute the starting point in the development comprising the present invention. The interchangeable terms "peptide fragment" and "peptide moiety" are meant to include both synthetic and naturally occurring amino acid sequences derivable from a naturally occurring amino acid sequence.

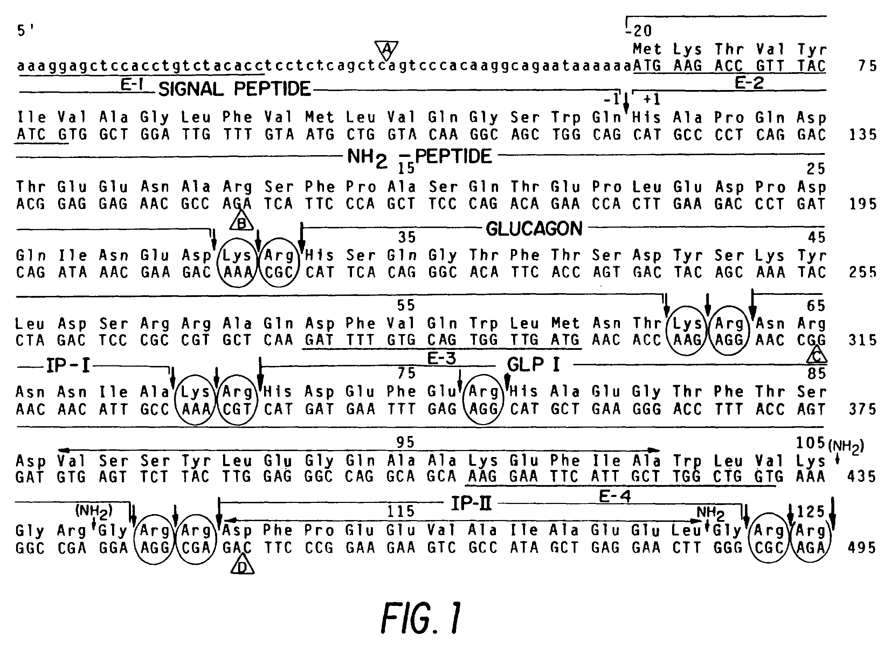

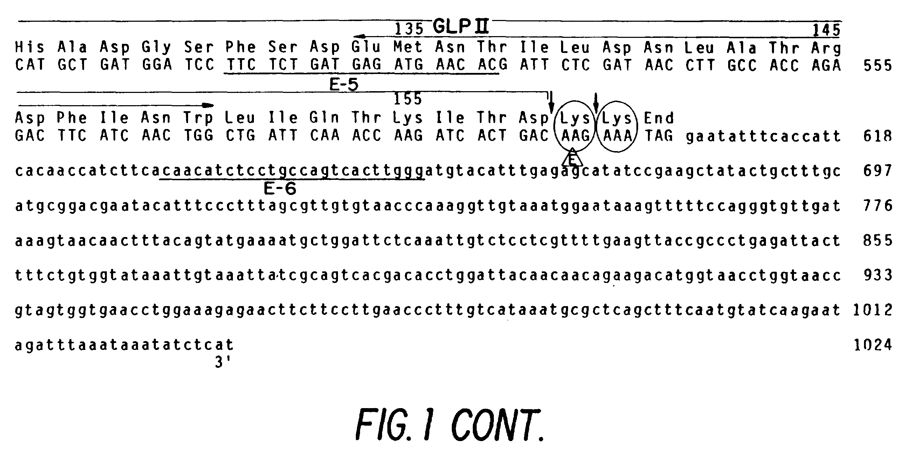

The amino acid sequence for GLP-1 has been reported by several researchers (Lopez, L.C., et al., Proc. Natl. Acad. Sci., USA 80:5485-5489 (1983); Bell, G.I., et al., Nature 302:716-718 (1983); Heinrich, G., et al., Endocrinol. 115:2176-2181 (1984); Ghiglione, M., et al., Diabetologia 27:599-600 (1984)). The structure of the preproglucagon gene and its corresponding amino acid sequence is shown in Figure 1. This figure further displays the proteolytic processing of the precursor gene product into glucagon and the two glucagon-like peptides. As used herein, the notation of GLP-1 (1-37) refers to a GLP-1 polypeptide having all amino acids from 1 (N-terminus) through 37 (C-terminus). Similarly, GLP-1 (7-37) refers to a GLP-1 polypeptide having all amino acids from 7 (N-terminus) through 37 (C-terminus).

In one embodiment, GLP-1 (7-37) and its peptide fragments are synthesized by conventional means, such as by the well-known solid-phase peptide synthesis described by Merrifield, J.M. (Chem. Soc. 85:2149 (1962)), and Stewart and Young (Solid Phase Peptide Synthesis (Freeman, San Francisco, 1969), pages 27-66), which are incorporated by reference herein. However, it is also possible to obtain fragments of the proglucagon polypeptide, or of GLP-1, by fragmenting the naturally occurring amino acid sequence, using, for example, a proteolytic enzyme. Further, it is possible to obtain the desired fragments of the proglucagon peptide or of GLP-1 through the use of recombinant DNA technology, as disclosed by Maniatis, T., et al., Molecular Biology: A Laboratory Manual, Cold Spring Harbor, New York (1982), which is hereby incorporated by reference.

The present invention includes peptides which are derivable from GLP-1 (1-37). A peptide is said to be "derivable from a naturally occurring amino acid sequence" if It can be obtained by fragmenting a naturally occurring sequence, or if it can be synthesized based upon a knowledge of the sequence of the naturally occurring amino acid sequence or of the genetic material (DNA or RNA) which encodes this sequence.

Included within the scope of the present invention are those molecules which are said to be "derivatives" of GLP-1 (1-37). Such a "derivative" has the following characteristics: (1) it shares substantial homology with GLP-1 (1-37) or a similarly sized fragment of GLP-1 (1-37); (2) it is capable of functioning as an insulinotropic hormone and (3) using at least one of the assays provided herein, the derivative has either (i) an insulinotropic activity which exceeds the insulinotropic activity of either GLP-1 (1-37) or GLP-1 (1-36), or, more preferably, (ii) an insulinotropic activity which can be detected even when the derivative is present at a concentration of 10⁻¹⁰ M, or, most preferably, (iii) an insulinotropic activity which can be detected even when the derivative is present at a concentration of 10⁻¹¹ M.

A derivative of GLP-1 (1-37) is said to share "substantial homology" with GLP-1 (1-37) if the amino acid sequences of the derivative is at least 80%, and more preferably at least 90%, and most preferably at least 95%, the same as that of either GLP-1 (1-37) or a fragment of GLP-1 (1-37) having the same number of amino acid residues as the derivative.

The derivatives of the present invention include GLP-1 (1-37) fragments which, in addition to containing a sequence that is substantially homologous to that of a naturally occurring GLP-1 (1-37) peptide may contain one or more additional amino acids at their amino and/or their carboxy termini. Thus, the invention pertains to polypeptide fragments of GLP-1 (1-37) that may contain one or more amino acids that may not be present In a naturally occurring GLP-1 (1-37) sequence provided that such polypeptides have an insulinotropic activity which exceeds that of GLP-1 (1-37) or GLP-1 (1-36).

Similarly, the invention includes GLP-1 (1-37) fragments which, although containing a sequence that is substantially homologous to that of a naturally occurring GLP-1 (1-37) peptide may lack one or more additional amino acids at their amino and/or their carboxy termini that are naturally found on a GLP-1 (1-37) peptide. Thus, the invention pertains to polypeptide fragments of GLP-1 (1-37) that may lack one or more amino acids that are normally present in a naturally occurring GLP-1 (1-37) sequence provided that such polypeptides have an insulinotropic activity which exceeds that of GLP-1 (1-37) or GLP-1 (1-36).

The invention also encompasses the obvious or trivial variants of the above-described fragments which have inconsequential amino acid substitutions (and thus have amino acid sequences which differ from that of the natural sequence) provided that such variants have an insulinotropic activity which is substantially identical to that of the above-described GLP-1 derivatives. Examples of obvious or trivial substitutions include the substitution of one basic residue for another (i.e. Arg for Lys), the substitution of one hydrophobic residue for another (i.e. Leu for Ile), or the substitution of one aromatic residue for another (i.e. Phe for Tyr), etc.

Examples of derivatives of GLP-1 (1-37) include GLP-1 (7-37); GLP-1 (7-36); GLP-1 (7-35); GLP-1 (7-34); and the des-Gly amidated forms of these molecules. Included as well are the use of additional amino acid residues added to such sequences in order to enhance coupling to carrier protein or amino acid residues added to enhance the insulinotropic effect.

As is known in the art, the amino acid residues may be in their protected or unprotected form, using appropriate amino or carboxyl protecting groups. Useful cations are alkali or alkaline earth metallic cations (i.e., Na, K, Li, 1/2Ca, 1/2Ba, etc.) or amine cations (i.e., tetraalkylammonium, trialkylammonium, where alkyl can be C₁-C₁₂).

The variable length peptides may be in the form of the free amines (on the N-terminus), or acid-addition salts thereof. Common acid addition salts are hydrohalic acid salts, i.e., HBr, HI, or, more preferably, HCl.

### B. Assays of Insulinotropic Activity

The present invention concerns GLP-1 (1-37) derivatives which have an insulinotropic activity that exceeds the insulinotropic activity of either GLP-1 (1-37) or GLP-1 (1-36). The insulinotropic property of a compound may be determined by providing that compound to animal cells, or injecting that compound into animals and monitoring the release of immunoreactive insulin (IRI) into the media or circulatory system of the animal, respectively. The presence of IRI is detected through the use of a radioimmunoassay which can specifically detect insulin. Although any radioimmunoassay capable of detecting the presence of IRI may be employed, It is preferable to use a modification of the assay method of Albano, J.D.M., et al., (Acta Endocrinol. 70:487-509 (1972)). In this modification, a phosphate/albumin buffer with a pH of 7.4 was employed. The incubation was prepared with the consecutive condition of 500 µl of phosphate buffer, 50 µl of perfusate sample or rat insulin standard in perfusate, 100 µl of anti-insulin antiserum (Wellcome Laboratories; 1:40,000 dilution), and 100 µl of [¹²⁵I] insulin, giving a total volume of 750 µl in a 10 x 75-mm disposable glass tube. After incubation for 2-3 days at 4°C, free insulin was separated from antibody-bound insulin by charcoal separation. The assay sensitivity was 1-2 µU/ml. In order to measure the release of IRI into the cell culture medium of cells grown in tissue culture, one preferably incorporates radioactive label into proinsulin. Although any radioactive label capable of labeling a polypeptide can be used, it is preferable to use ³H leucine in order to obtain labeling proinsulin. Labeling can be done for any period of time sufficient to permit the formation of a detectably labeled pool of proinsulin molecules; however, it is preferable to incubate cells in the presence of radioactive label for a 60-minute time period. Although any cell line capable of expressing insulin can be used for determining whether a compound has an insulinotropic effect, it is preferable to use rat insulinoma cells, and especially RIN-38 rat insulinoma cells. Such cells can be grown in any suitable medium; however, it is preferable to use DME medium containing 0.1% BSA and 25 mM glucose.

The insulinotropic property of a compound may also be determined by pancreatic infusion. The in situ isolated perfused rat pancreas preparation was a modification of the method of Penhos, J.C., et al. (Diabetes 18:733-738 (1969)). In accordance with such a method, fasted rats (preferably male Charles River strain albino rats), weighing 350-600 g, are anesthetized with an intraperitoneal injection of Amytal Sodium (Eli Lilly and Co., 160 ng/kg). Renal, adrenal, gastric, and lower colonic blood vessels are ligated. The entire intestine is resected except for about four cm of duodenum and the descending colon and rectum. Therefore, only a small part of the intestine is perfused, thus minimizing possible interference by enteric substances with glucagon-like immunoreactivity. The perfusate is preferably a modified Krebs-Ringer bicarbonate buffer with 4% dextran T70 and 0.2% bovine serum albumin (fraction V), and is preferably bubbled with 95% O₂ and 5% CO₂. A nonpulsatile flow, four-channel roller-bearing pump (Buchler polystatic, Buchler Instruments Division, Nuclear-Chicago Corp.) is preferably used, and a switch from one perfusate source to another is preferably accomplished by switching a three-way stopcock. The manner in which perfusion is performed, modified, and analyzed preferably follows the methods of Weir, G.C., et al., (J. Clin. Investigat. 54:1403-1412 (1974)), which are hereby incorporated by reference.

### C. Formulations of Insulinotropic Compounds

The insulinotropic peptides (or peptide derivatives) of GLP-1 (1-37) may be used as therapeutic compositions. Such therapeutic compositions may consist solely of the insulinotropic peptides (or peptide derivatives) although, preferably, the compositions will contain the insulinotropic peptides (or derivatives thereof) combined in admixture with a pharmaceutically acceptable carrier vehicle.

Suitable vehicles and their formulation, inclusive of other human proteins, e.g., human serum albumin, are described for example in Remington's Pharmaceutical Sciences (16th Ed., A. Oslo Ed. Mack, Easton, PA (1980)). In order to form a pharmaceutically acceptable composition suitable for effective administration, such compositions will contain an effective amount of GLP-1 (7-37), or a derivative of GLP-1 (7-37), together with a suitable amount of carrier vehicle. The GLP-1 derivatives of such compounds will preferably have been purified so as to be substantially free of natural contaminants. A material is said to be "substantially free of natural contaminants" if it has been substantially purified from materials with which it is normally and naturally found. Examples of natural contaminants with which GLP-1 (7-37) might be associated are: other peptides, carbohydrates, glycosylated peptides, lipids, membranes, etc. A material is also said to be substantially free of natural contaminants if these contaminants are substantially absent from a sample of the material.

Compositions containing GLP-1 (7-37) or its derivatives may be administered intravenously, intramuscularly, or subcutaneously at dosages in the range of from about 1 pg/kg to 1,000 µg/kg body weight, or at concentrations sufficient to produce serum levels of 10⁻¹⁰ M to 10⁻¹¹ M, although a lower or higher dosage may be administered. The required dosage will depend upon the severity of the condition of the patient and upon such criteria as the patient's height, weight, sex, age, and medical history.

For the purpose of parenteral administration, compositions containing the derivatives of GLP-1 (1-37) are preferably dissolved in distilled water and the pH-value is preferably adjusted to about 6 to 8. In order to facilitate the lyophilization process resulting in a suitable product, lactose may be added to the solution. Preferably, the solution is then filtered sterilized, introduced into vials, and lyophilized. The concentration of the GLP-1 (1-37) derivatives in these compositions may vary from 10⁻¹²M to 10⁻⁵M.

Additional pharmaceutical methods may be employed to control the duration of action. Controlled release preparations may be achieved by the use of polymers to complex or adsorb the GLP-1 (1-37) derivatives. The controlled delivery may be exercised by selecting appropriate macromolecules (for example, polyesters, polyamino acids, polyvinyl pyrrolidone, ethylenevinylacetate, methylcellulose, carboxymethylcellulose, and protamine sulfate) and the concentration of macromolecules as well as the methods of incorporation in order to control release. Another possible method to control the duration of action by controlled release preparations is to incorporate the derivatives of GLP-1 (1-37) into particles of a polymeric material such as polyesters, polyamino acids, hydrogels, poly (lactic acid) or ethylene vinyl-acetate copolymers. Alternatively, instead of incorporating the GLP-1 (1-37) derivatives into these polymeric particles, it is possible to entrap these derivatives in microcapsules prepared, for example, by coacervation techniques or by interfacial polymerization, for example, hydroxymethylcellulose or gelatin-microcapsules and poly (methylmethacrylate) microcapsules, respectively, or in colloidal drug delivery systems, for example, liposomes, albumin microspheres, microemulsions, nanoparticles, and nanocapsules or in macroemulsions. Such teachings are disclosed in Remington's Pharmaceutical Sciences (1980).

It is possible to enhance the biological half-life of the GLP-1 (1-37) derivatives of the present invention, and, thus, to increase the retention or stability of the derivatives in a recipient, by bonding such derivatives to one or more chemical "moieties" to thereby produce a compound which can be recognized and processed within a recipient to yield a GLP-1 (1-37) derivative. The "moieties" of such compounds may include one or more lipids, carbohydrates, amino acid residues, etc. A preferred "moiety" is an amino acid residue. The most preferred "moiety" is a peptide. The amino terminal (histidine) residue of GLP-1 (7-37) is a preferred site for the bonding of the "moiety".

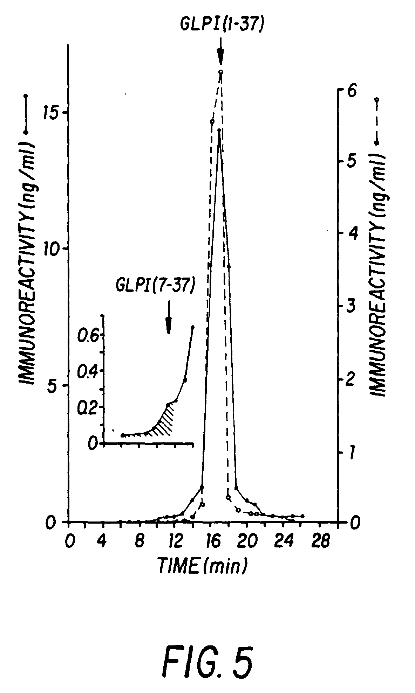

An appreciation of this aspect of the present invention can be obtained through a consideration of the natural processing of GLP-1 (1-37). GLP-1 (1-37) has a biological half-life of 30-50 minutes. A natural cleavage of the amino terminal hexapeptide, GLP-1 (1-6), occurs to yield GLP-1 (7-37) whose biological half-life is only 3-5 minutes. Thus, the amino terminal hexapeptide, GLP-1 (1-6) is a natural "moiety" which when bonded to GLP-1 (7-37) increases the biological half-life of GLP-1 (7-37). The discovery of such a natural "moiety" is disclosed in Figure 5, and supports the concept that additional or alternative moieties may be employed in the same manner as GLP-1 (1-6) to increase the biological half-life of the GLP-1 (1-37) derivatives of the present invention. Although the present invention does not encompass the use of GLP-1 (1-6) as a "moiety," it does include variants of GLP-1 (1-6) as well as other peptides of unrelated sequence which are capable of enhancing the half-life of the peptides and peptide derivatives of the present invention.

In summary, insulin secretion from the β-cell of the endocrine pancreas is controlled by a complex network of metabolic factors. This network includes such diverse components as glucose, amino acids, catecholamines, and peptides. The decoding of the glucagon gene has uncovered two additional glucagon-like peptides encoded in proglucagon, the polypeptide precursor of glucagon. One of these peptides, glucagon-like peptide-1 (GLP-1) is processed from proglucagon in two forms: 37-amino acids GLP-1 (1-37) and 31-amino acid GLP-1 (7-37). The specific liberation of GLP-1 peptide's in the intestine and, to some degree, in the pancreas, suggested to the inventors that the GLP-1 peptide's might be components of the entero-insular axis. To resolve this issue, the effects of the GLP-1 peptide's on a β-cell line was studied using a rat perfused pancreas and a cultured rat insulinoma cell-line. These studies have revealed that, in the isolated perfused pancreas, GLP-1 (7-37) is a potent stimulator of insulin secretion at concentrations as low as 5 X 10⁻¹²M. Insulin release in response to GLP-1 (7-37) is highly dependent upon ambient glucose concentration. The longer peptide (GLP-1 (1-37)) has no insulin-releasing activity even at concentrations as high as 5 X 10⁻⁷M. Comparison of the insulinotropic effects of GLP-1 (7-37) and glucagon showed that (in the rat perfused pancreas) GLP-1 (7-37) is at least 100 fold more potent in the stimulation of insulin sectretion. In the rat insulinoma cell line (RIN 1046-38) GLP-1 (7-37), at concentrations of 10⁻¹⁰M to 10⁻¹¹M, increased both the cellular levels of cAMP (5-fold) and the levels of insulin mRNA (3-fold) and also stimulated insulin release. Again the effects of GLP-1 (7-37) were more potent than those of glucagon. The magnitude of the insulinotropic effects at such low concentrations renders GLP-1 (7-37) one of the most potent insulin secretagogues described, including glucagon and gastric inhibitory polypeptide. These results suggest that GLP-1 (7-37) may participate in the physiological regulation of β-cell functions.

Having now fully described the invention, the same will be more readily understood by reference to specific examples which are provided by way of illustration, and are not intented to be limiting of the invention, unless specified.

### SPECIFIC EXAMPLES

### EXAMPLE 1

### Specificity of GLP-1 Peptides

In order to demonstrate that the effects of GLP-1 (1-37), GLP-1 (1-36) and GLP-1 (7-37) were specific for insulin, and were not capable of inducing or provoking non-specific gene expression, the effect of these peptides on the levels of insulin, actin and angiotensinogen mRNAs in rat insulinoma cells were conducted.

Rat insulinoma cells of cell line RIN-38 were derived from a continuous islet cell line, RIN-r, which was established from a transplantable rat islet cell tumor (Gazdar, A.F., et al., Proc. Natl. Acad. Sci., USA 77:3519-3523 (1980)). The cells were maintained in DMEM (Gibco) at a glucose concentration of 4,500 mg/L and supplemented with 10% heat-inactivated fetal bovine serum (Gibco), 100 U/ml of penicillin and 100 µg/ml of streptomycin. Incubations were carried out at 37°C in 95% air:5% CO₂. Cells grown in the above manner were washed and resuspended in DMEM (Gibco) containing 0.1% bovine serum albumin and 25 mM glucose. Cells were incubated with varying concentrations of insulinotropic peptides (i.e. glucagon, GLP-1 (1-37), GLP-1 (7-37), or GLP-1 (1-36 des-gly-arg amide); Peninsula Laboratories) for six hours, following which the effects of these agents on mRNA expression were determined. In all cases, the concentration of peptides was 10⁻⁷M. Incubations were for six hours.

Messenger RNAs specific for insulin, actin, or angiotensinogen were identified by Northern hybridization as follows: cellular RNA was extracted from solid tumors and cells by homogenization in guanidine thiocyanate and sedimentation through a cesium chloride cushion. Poly A⁺ RNA was isolated by oligo dT cellulose chromatography (Aviv, H., et al., Proc. Natl. Acad. Sci., USA 69:1408-1412 (1972)). Twenty micrograms of total RNA from each sample were fractionated by size on a 1.4% agarose gel after denaturation in glyoxal, followed by electrotransfer to a nylon membrane (Nytran; Schleicher and Schuell). Blotted membranes were baked for two hours at 80°C under vacuum, prehybridized in 1M NaCl/1% SDS/10% Dextran sulfate at 50°C overnight and hybridized at the same temperature for 24 h after addition of the labeled probes (3-5 x 10⁵ cpm/ml); they were then washed at 55°C twice in 1 x SCC (0.15M NaCl/0.015M Na citrate)/1% SDS), and exposed to X-ray film for varying times at -70°C with an intensifying screen. The relative amounts of the specific mRNA were determined by microdensitometry. The results of this experiment are shown in Table 1.

The glucagon-like peptides increased the levels of insulin mRNA during 24-hr incubations (Table 1). The increase in insulin mRNA levels was consistently greater in response to the shorter, 31-amino acid peptide; 3-fold higher than control values at 24 hr. These stimulatory effects on insulin mRNA levels and on the release of insulin were observed in the presence of high (25 mM) and not low (5.5 mM) concentrations of glucose. Evidence that the stimulatory actions of GLP-1 are relatively specific for insulin mRNA was obtained by demonstrating that (i) GLP-1 (7-37) had negligible effects on levels of actin and angiotensinogen mRNAs in the insulinoma cell line; (ii) glucagon and GLP-1I had no effects on insulin mRNA levels; and (iii) GLP-1 (7-37), when added to the rat islet glucagon-producing cell line 1056A and two pituitary cell lines, one producing prolactin (GH4) and the other corticotropin (AtT-20), had no effects on the levels of glucagon, prolactin, and corticotropin mRNAs, respectively.

GLP-1 (1-37) was examined to determine whether it could induce the biosynthesis of mRNA of hormones other than insulin. Thus, GLP-1 (1-37) (at a concentration of 10⁻⁷M) was added to a rat islet glucagon-producing cell line and two pituitary cell lines (GH4 and AtT-20) which were capable of producing the hormones prolactin and ACTH, respectively, and the amount of hormone specific mRNA produced was determined after 24 hours as described above. GLP-1 peptides had no detectable effect on either the level of prolactin mRNA in GH4 pituitary cells, or in the level of ACTH mRNA in AtT-20 pituitary cells.

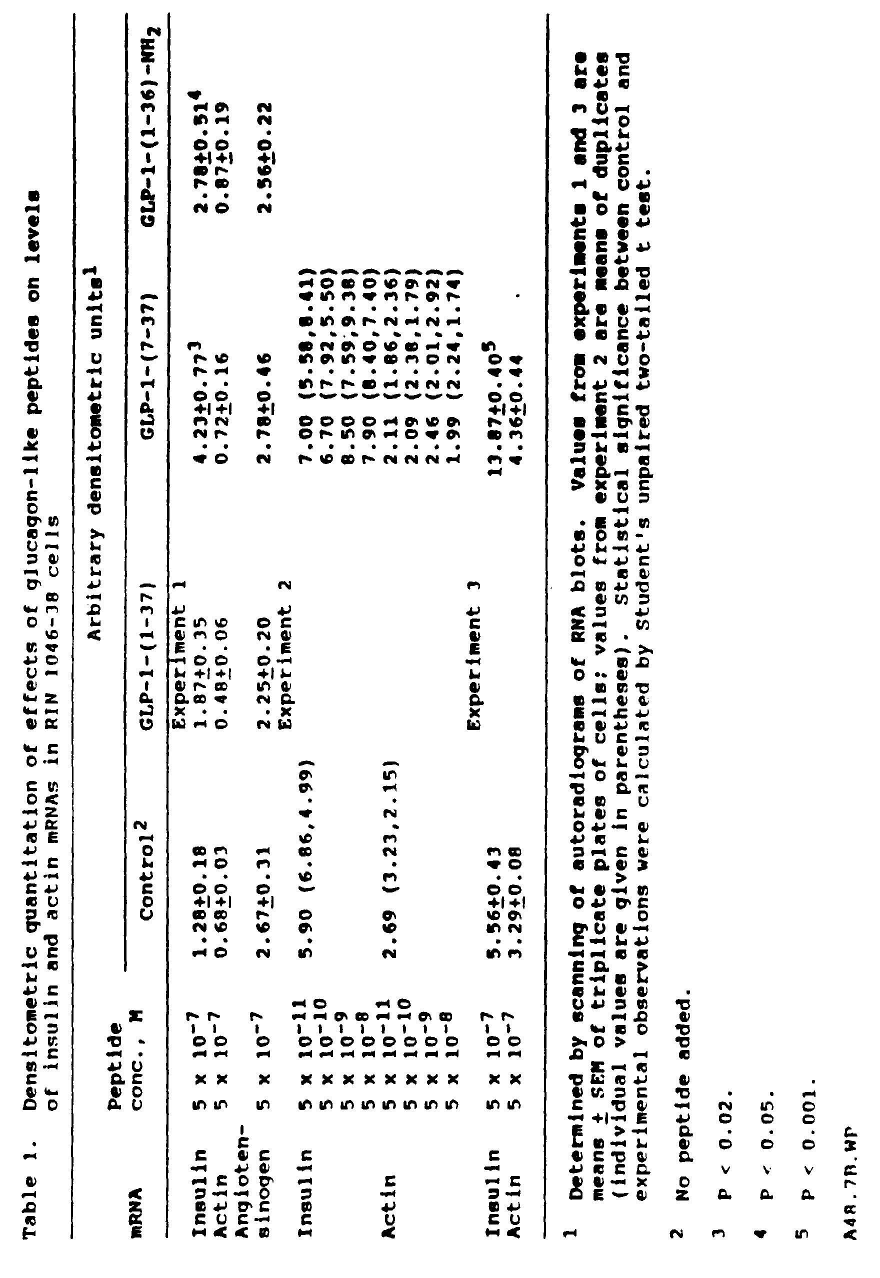

### EXAMPLE 2

### The Effect of GLP-1 (7-37) on the Transcription of the Insulin and Other Genes

The effect of GLP-1 (7-37) on the transcription of the insulin and actin genes in RIN-38 insulinoma cells was investigated. Gene transcription rates were determined by quantification of nascent insulin and beta-actin RNA transcripts in nuclei from control and GLP 7-37 treated cells. The GLP-1 (7-37) concentration was 10⁻⁷M. Incubation was for 4 hours. Nuclear RNA was hybridized to an excess of cloned specific DNA bound to nitrocellulose and the filters were washed as described by McKnight, G.S., et al., ( J. Biol. Chem. 254:9050-9058 (1979)). Rat insulin (Ullrich, A., et al., Science 196:113-119 (1977)) and, for control, chicken beta-actin cDNAs, provided by Dr. D. Cleveland, the Johns Hopkins University School of Medicine, Baltimore, Maryland, were used. Hybridization efficiency was controlled through the addition of the hybridization solution of [³H] UTP insulin cRNA. Experiments were done in duplicate and values are expressed in ppm/kb of cDNA insert, corrected for efficiency of hybridization (40-50%). The results of this experiment revealed that GLP-1 (7-37) increased the rate of insulin gene transcription, but had no detectable effect upon the rate of actin gene transcription.

### EXAMPLE 3

### Effect of GLP-1 Derivatives on Cellular cAMP Levels

In order to determine whether glucagon-like proteins were capable of affecting cellular cAMP levels, the effects of GLP-1 (7-37) and GLP-1 (1-37) on cAMP levels in RINS-38 insulinoma cells (Expt. I and Expt. II, respectively) was determined.

Cells were grown as described in Example 1, in 26 well culture dishes. Varying amounts of glucagon-like peptides were added to culture wells in triplicate. After permitting incubation for 10 minutes, the total cell media was examined for cAMP, and the concentration of cAMP was determined. The results of this experiment are shown in Table 2. Twenty microliters from each culture well was assayed.

**Table 2**

| Peptide Concentration (M) | Expt. I | Expt II |
|---|---|---|
| 0 | 140 | 91 |
| 10⁻⁶ | 400 | 170 |
| 10⁻⁷ | 370 | 120 |
| 10⁻⁸ | 494 | 160 |
| 10⁻⁹ | 515 | 100 |
| 10⁻¹⁰ | 253 | 90 |
| 10⁻¹¹ | 533 | 90 |

This experiment reveals that GLP-1 (7-37) was capable of stimulating cAMP levels even when present at a concentration of 10⁻¹¹M. The increase in cAMP levels is an indication that GLP-1 (7-37) is capable of interacting with cellular receptors. In contrast, neither GLP-1 (1-37) nor GLP-II exhibited such activity.

A further experiment was performed in order to compare the insulinotropic activities of GLP-1 (1-37), GLP-1-(1-36)-NH₂ and GLP-1 (7-37) with the insulinotropic activity of glucagon. The procedures for this experiment are the same as those described above. The results of this experiment are shown in Table 3.

At the relatively high concentration of 0.5 µM, GLP-1 (1-37), GLP-1-(1-36)-NH₂, GLP-1 (7-37) and glucagon each increased cAMP levels. At 5 nM, GLP-1-(7-37) increased cAMP levels at least 4-fold and was still active at 50 pM. In contrast, the effects of glucagon, GLP-1-(1-37), and GLP-1-(1-36)-NH₂ on the formation of cAMP were negligible at these concentrations.

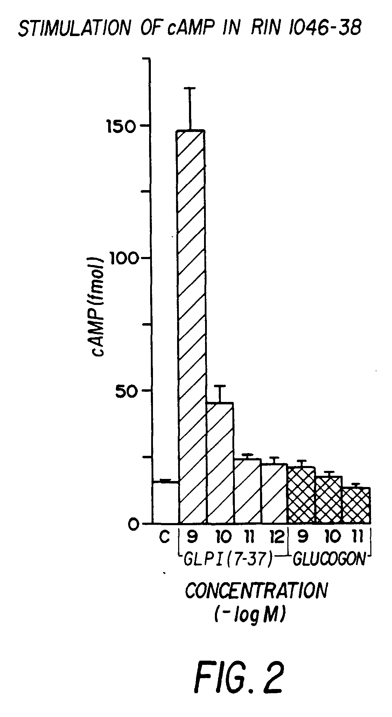

The ability of the insulinotropic peptides glucagon and GLP-1 (7-37) to stimulate cAMP formation in the insulinoma line, RIN 1046-38, was investigated. Insulinotropic activity was monitored as described by Mojsov, S., et al., (J. Clin. Invest. 79:616-619 (1987)) and Drucker, D.J., et al., (Proc. Natl. Acad. Sci. 84:3434-3438 (1987)), both of which references are incorporated by reference herein. The results of this study are shown in Figure 2, and indicate that GLP-1 (7-37) is at least 1000 times more potent than glucagon in inducing cAMP formation.

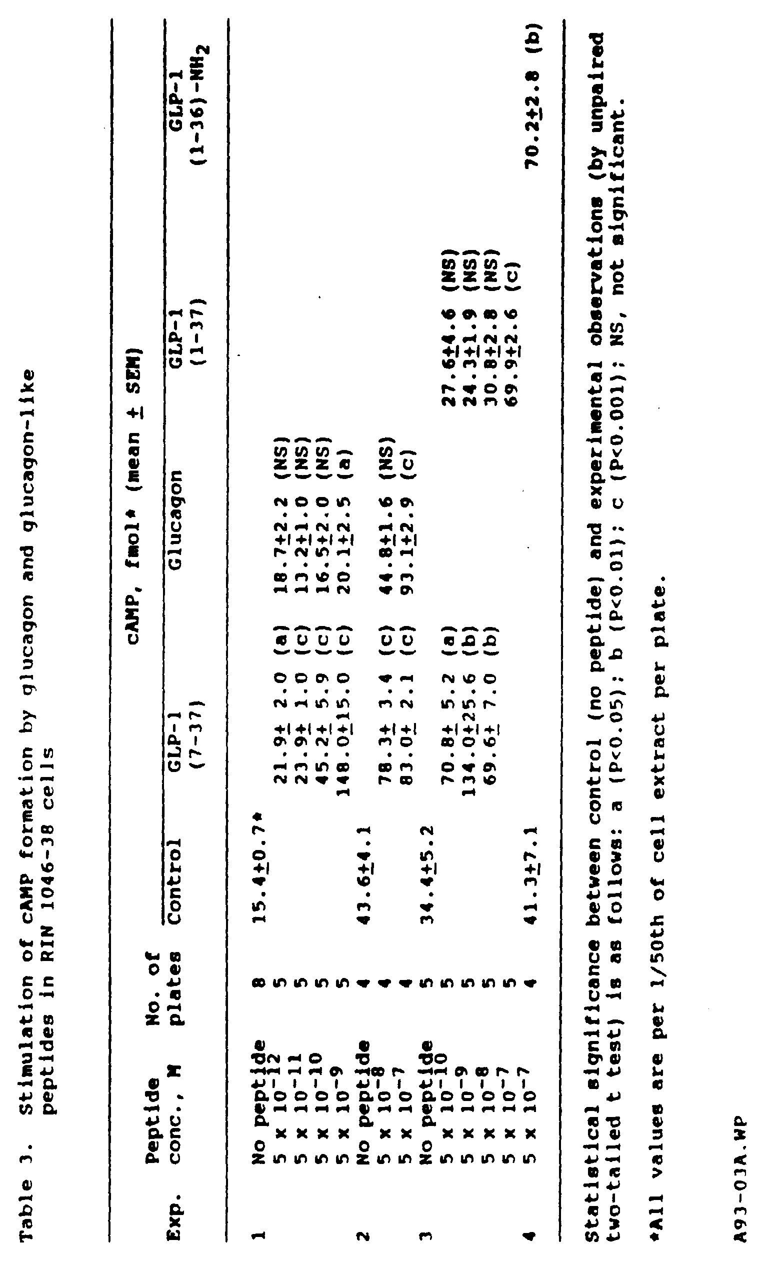

### EXAMPLE 4

### Effect of GLP-1 Peptides on Insulin Production

Rat insulinoma cells of cell line RIN-38 were grown in DME medium as described in Example 1. After incubation with 5 x 10⁻⁷M GLP-1 (7-37), the concentrations of insulin in the cell culture mediums were determined by radio-immunoassay (as described above). Insulin protein levels were determined after incubation for 1 or 24 hours. The results of this experiment are shown in Table 4.

**Table 4**

| | Insulin Produced (µunits/ML) | |
|---|---|---|
| Peptide Added | 1 Hour | 24 Hours |
| None | 166 | 2,778 |
| GLP-7 (7-37) | 381 | 5,164 |

### EXAMPLE 5

### Pancreatic Perfusion Assay of Insulinotropic Activity

The pancreas of live rat was perfused with varying concentrations of GLP-1 (1-37) and GLP-1 (7-37) as described above. Isolated rat pancreas was prepared according to the method of Weir, G.C., et al., (J. Clin. Invest. 54:1403-1412 (1974)), and Penkos, J.C., et al. (Diabetes 18:733-738 (1969)). The perfusate contained bicarbonate buffer (pH 7.4) and 120 mg/dl glucose, 4% dextran T-70, and 0.2% bovine serum albumin, and was equilibrated with 95% oxygen and 5% carbon dioxide. The first 20 minutes of each perfusion was an equilibrium period. After this initial period, aliquots of perfusate were removed every 2-4 min for additional 20 min, thus allowing the system to equilibrate for a total of 40 min. The perfusion, including any added insulinotropic peptide, was for 6 min and samples were collected at 1-min intervals. When more than one perfusion was to be performed, the peptide perfusions were followed by equilibration periods of 20 min, during which four samples 5 min apart were collected. A second 6-min perfusion followed with the same peptide as the first perfusion only at 100 times higher concentration of peptide. Again, samples 1 min apart were collected. The entire perfusion time was between 70 and 85 mm.

In each aliquot of perfusate obtained, insulin was determined by radioimmunoassay. In addition, the efficiency of delivery of the insulinotropic peptide was confirmed by radioimmunoassay of corresponding aliquots of perfusate in which insulin was measured (Mojsov, S.G., et al., J. Biol. Chem. 261:11880-11889 (1986), which reference is incorporated herein by reference). At one minute intervals, rat serum insulin levels in picograms/ml were determined by radioimmunoassay (as described above). The results of this experiment are shown in Table 5. Perfusions were done using peptide concentrations of 5 x 10⁻⁷M, 5 x 10⁻⁸M, and 5 x 10⁻¹⁰M, 5 x 10⁻¹¹M, and 5 x ⁻¹²M. Peptides were added after the zero minute serum value had been determined.

GLP-1 (1-37) was found to mediate a 3.4-fold increase in serum insulin concentrations when perfused into rat pancreas at a concentration of 5 x 10⁻⁷M; at a concentration of 5 x 10⁻⁸M, this peptide was capable of mediating only a two-fold increase in serum insulin levels. At a concentration of 5 x 10⁻¹⁰M, this peptide was found to mediate only a 20% increase in serum insulin levels. The observed insulinotropic activity of GLP-1 (1-37) in these experiments most probably reflects the presence of GLP-1 (7-37) in the preparations ( either through the degradation of GLP-1 (1-37) or due to low level contamination).

GLP-1 (7-37) was found to be capable of stimulating a 132-fold increase in insulin levels when provided to rat pancreas at a concentration of 5 x 10⁻⁷M. At a 10-fold lower concentration (5 x 10⁻⁸), this peptide was capable of directing a 21-fold increase in the serum concentration of insulin. At a concentration of 5 x 10⁻¹⁰M, GLP-1 (7-37) was found to be capable of mediating an increase in serum insulin levels (32-fold). Even at a concentration of 5 x 10⁻¹¹M, GLP-1 (7-37) delivered a 15-fold increase in insulin levels whereas GLP-1 (1-37) was without effect.

This experiment shows that GLP-1 (7-37) is more than 1,000-fold more potent than GLP-1 (1-37) in stimulating insulin expression in vivo. In addition, the GLP-1 peptides had no effects on the release of the peptide hormones glucagon and somatostatin in these same experiments. Thus, the stimulatory effects of GLP-1 are specific for the beta cells and do not act on pancreatic alpha or delta cells.

The level of GLP-1 (1-37) and GLP-1 (7-37) in rat portal blood has been measured by radioimmunoassay to be approximately 150 pg/ml (50 pM). The corresponding level in peripheral blood is 50 pg/ml (15 pM). The above-described results were obtained using GLP-1 (7-37) at a concentration of 5-50 pM. Thus, these results indicate that GLP-1 (7-37) has insulinotropic activity at its physiologic concentration.

**Table 5**

| Insulin Produced (picograms/ml) at Peptide Concentration | | | | | | |
|---|---|---|---|---|---|---|
| | Minutes | 5x10⁻⁷M | 5x10⁻⁸M | 5x10⁻¹⁰M | 5x10⁻¹¹M | 5x10⁻¹²M |
| GLP-1 (7-37) | 0 | 50 | 925 | 205 | 160 | 50 |
| | 1 | 6,600 | 20,700 | 7,400 | 2,400 | |
| | 50 | | | | | |
| | 2 | 4,700 | 10,500 | 1,800 | 1,700 | |
| | 50 | | | | | |
| | 3 | 1,700 | 4,000 | 760 | 1,900 | |
| | 98 | | | | | |
| GLP-1 (1-37) | 0 | 1,400 | 3,000 | 500 | 340 | |
| | 50 | | | | | |
| | 1 | 4,700 | 6,000 | 600 | 180 | |
| | 50 | | | | | |
| | 2 | 2,900 | 2,000 | 640 | 230 | |
| | 160 | | | | | |
| | 3 | 2,200 | 2,000 | 430 | 340 | 50 |

### EXAMPLE 6

### Comparison of the Insulinotropic Activities of Glucagon and GLP-1 (7-37)

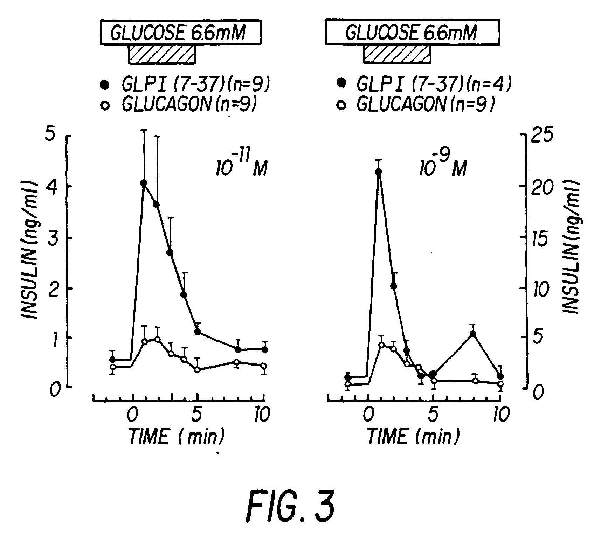

Rat pancreas perfusion experiments were conducted as described in Example 6, in order to compare the insulinotropic activity of glucagon with that of GLP-1 (7-37). Peptides were perfused (for 5 minutes) at concentrations of 10⁻⁹M and 10⁻¹¹M. As shown in Figure 3, GLP-1 (7-37) was found to have more than 100 times the insulinotropic activity of glucagon.

This finding is confirmed by the study of the effects of glucagon, GLP-1 (1-37), and GLP-1 (7-37) on the cAMP levels in RIN 1046-38 insulinoma cells which is presented in Table 3.

### EXAMPLE 7

### Insulinotropic Activity of Derivatives of GLP-1 (1-37)

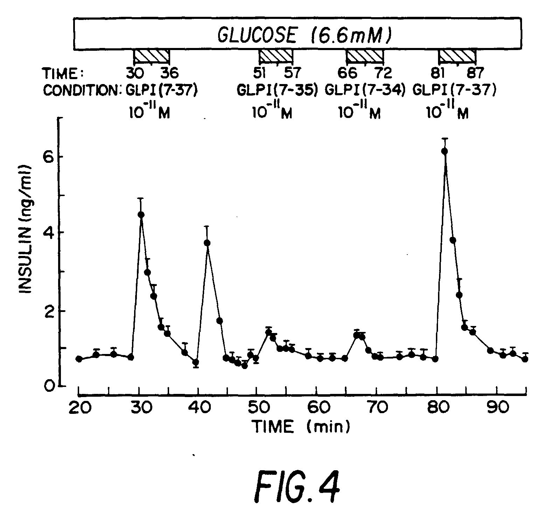

The insulinotropic activities of GLP-1 (7-34) and GLP-1 (7-35) were compared to that of GLP-1 (7-37) using the rat pancreas perfusion technique described above. Five minute perfusions were employed. As shown in Figure 4 all three of these peptides had detectable insulinotropic activity at a concentration of 10⁻¹¹ M. These results indicate that the tested derivatives of GLP-1 (7-37) all have an insulinotropic activity greater than that of GLP-1 (1-37, which is inactive at 10⁻¹¹ M.

The GLP-1 related peptides: GLP-1 (7-36)-NH₂ and GLP-1 (7-37) were compared to determine their relative insulinotropic activities. Insulinotropic activity was determined using the pancreatic perfusion assay of Example 6; the perfusion being carried out for 5 minutes. Peptides were present at a concentration of 10⁻¹¹ M. The results of this experiment are shown in Table 6. These results indicate that both GLP-1 (7-37) and GLP-1 (7-36)-NH₂ have substantial insulinotropic activity.

**TABLE 6**

| Comparison of the Insulinotropic Activity of GLP-1 (7-37) and GLP-1 (7-36)-NH₂ | | |
|---|---|---|
| Time (Minutes) | GLP-1 (7-36)-NH₂ | GLP-1 (7-37) |
| -1 | 985 ± 275 | 1330 ± 280 |
| +1 | 4550 ± 703 | 4200 ± 784 |
| +2 | 3330 ± 637 | 3280 ± 889 |
| +3 | 2500 ± 564 | 2500 ± 505 |

The effects of GLP-1 (7-37) and GLP-1 (7-36)-NH₂ on cAMP formation in the insulinoma cell line RIN 1046-38 was determined using the procedure of Example 1. Cells were incubated for 10 minutes in the presence of 0, 10⁻¹¹, 10⁻⁹, or 10⁻⁷M peptides. The results of this experiment are shown in Table 7. These results confirm that both GLP-1 (7-37) and GLP-1 (7-36)-NH₂ are insulinotropic peptides.

**TABLE 7**

| EFFECTS OF GLP-1 (7-37) VERSUS GLP-1 (7-36)NH₂ ON cAMP FORMATION IN AN INSULINOMA CELL LINE (RIN 1046-38 CELLS). | | | | | | |
|---|---|---|---|---|---|---|
| | | | | Peptide Concentration (M) | | |
| Peptide | N | IBMX (uM) | 0 | 10⁻¹¹ | 10⁻⁹ | 10⁻⁷ |
| None (Control) | 8 | 100 | 161±10* | | | |
| GLP-1 (7-37) | 4 | 100 | | 205±9 | 202±17 | 317±65 |
| GLP-1 (7-36)NH₂ | 4 | 100 | | 141±5 | 225±16 | 358±32 |
| None (Control) | 8 | 500 | 540±22 | | | |
| GLP-1 (7-37) | 4 | 500 | | 501±49 | 927±75 | 2114±421 |
| GLP-1 (7-36)NH₂ | 4 | 500 | | 446+38 | 1199+41 | 1676+113 |

| | | | | | | |
|---|---|---|---|---|---|---|
| *All cAMP values are given as/fmoles/10ul of cell extract | | | | | | |

### EXAMPLE 8

### Stability of GLP-1 (1-37)

To assess the stability of the 31 amino acid peptide in the experimental conditions, GLP-1 (1-37) was incubated for 24 hr in culture medium alone or in medium supplemented with either 0.1% bovine serum albumin or 10% fetal bovine serum. Aliquots of media were analyzed by high-pressure liquid chromatography and radioimmunoassay. Before incubation, no GLP-1 (7-37) was detected in the preparation of GLP-1 (1-37) (Figure 5). However, after incubation of GLP-1 (1-37) in conditioned medium containing 01% bovine serum albumin, a small peak of GLP-1 (7-37) appeared, Indicating that cleavage of GLP-1 (1-37) to the smaller, more active GLP-1 (7-37) occurs under these experimental conditions.

### EXAMPLE 9

### Insulinotropic Effect of GLP-1 (8-37)

As discussed above, glucagon has been found to be a beta-cell secretagogue, acting at concentrations as low as 10⁻⁹ M. GLP-1 (7-37), which is co-encoded on the preproglucagon gene, has, as shown above, the capacity to mediate an insulinotropic effect even at concentrations as low as 10⁻¹² M. In order to determine if separate receptors might be involved in the recognition of glucagon and GLP-1 (7-31), a potential GLP-1 antagonist, the analog des-7 histidine GLP-1 (7-37) was constructed. This analog is hereinafter referred to "GLP-1 (8-37)".

Insulin secretion was studied in the perfused rat pancreas assay described above, with a perfusate glucose level of 6.6 mM. GLP-1 (8-37) was found to have no detectable effect at concentrations at 10⁻¹¹, 10⁻⁹, or 10⁻⁸ M. A weak insulinotropic activity was detected at 10⁻⁷ M. At a perfusate glucose level of 16.7 mM the analog had no effect at 10⁻⁹ M.

A similar experiment was conducted using glucagon. Glucagon was infused into rat pancreas for 5 minutes at a concentration of 10⁻⁹ M with a perfusate glucose level of 6.6 mM, in either the presence or absence of 10⁻⁸ M GLP-1 (8-37). Glucagon was found to elicit an elevated mean perfusate insulin concentration of 4.98 ± 0.96 ng/ml, and virtually identical results (5.26 ± 0.89 ng/ml) were seen with glucagon in the presence of GLP-1 (8-37) (N=4).

The above described protocol was used to study the effects of the GLP-1 (7-37) in the presence or absence of GLP-1 (8-37). GLP-1 (7-37), alone at a concentration of 10⁻¹¹ M stimulated mean insulin release of 2.25 ± 0.30 ng/ml. This response was, however, lower (1.30 ± 0.19 ng/ml (P < 0.025, N=7 for each)) when the same dose was given on a background of the analog. These data indicate that the removal of the 7-histidine from GLP-1 (7-37) leads to a loss of agonist function in this system, and that agonist properties are revealed. Because the agonist activity could only be demonstrated against GLP-1 (7-37), and not against glucagon, these two secretagogue appear to act through separate receptors.

Using the above-described perfused rat pancreas system, and a glucose perfusate of 6.6 mM, it was found that 10⁻⁹ M GLP-1 (7-37) was capable of eliciting a biphasic pattern of insulin secretion with an initial spike of release followed by the plateau of sustained release. Furthermore, this response was found to be glucose-dependent; at 10⁻⁹ M and a perfusate glucose concentration of 2.8 mM, no stimulation of insulin release was seen; at a perfusate glucose concentration of 6.6 mM or 16.7 mM, the mean incremental release above control was 4.7 ± 1.0 or 22.8 ± 4.7 ng/ml, respectively. GLP-1 (7-37) was found to be extraordinarily potent. At concentrations of 10⁻¹² M it was found to stimulate insulin secretion at a perfusate glucose concentration of 6.6 mM from a base line of 0.8 ± 0.2 ng/ml to a peak of 1.6 ± 0.5 ng/ml (P < 0.05). When infused at 10⁻¹¹ M, insulin release was stimulated to a peak of 4.1 ± 1.4 ng/ml and at 10⁻⁹ M, a peak of 23.1 ± 1.3 ng/ml was obtained. It is not yet known whether this GLP-1 peptide is secreted as the 7-37 form or is the 7-36-amide; both compounds were equally potent secretagogues.

Synthetic glucagon was far less potent with no release found at 10⁻¹¹ M. At 10⁻⁹ M, however, glucagon was found to produce a peak of 4.5 ± 0.5 ng/ml.

Thus, GLP-1 (7-37) and the 7-36-amide are candidates for physiological regulation as an "incretin" or as and endocrine modulator.

### EXAMPLE 10

### Affects of GLP-1 (8-37) on cAMP Formation in an Insulinomina Cell Line

A comparison of the insulinotropic effects of GLP-1 (8-37), GLP-1 (7-37) and glucagon on cAMP formation by the insulinoma cell line RIN 1046-38 was determined using the procedure of Example 1. The results of this experiment is shown in Table 8. These results show that GLP-1 (8-37) has an insulinotropic activity which is comparable to that of glucagon, and that GLP-1 (7-37) has an insulinotropic activity which is more than 100 times greater than that of either GLP-1 (8-37) or glucagon.

**TABLE 8**

| Effects of GLP-1 (8-37) versus GLP-1 (7-37) and Glucagon on cAMP Formation in an Insulinoma Cell Line (RIN 1046-38 cells) | | |
|---|---|---|
| Peptide | Concentration (M) | cAMP Formation* |
| None | | 27 ± 2 |
| GLP-1 (8-37) | 10⁻⁸ | 28 ± 0.8** |
| | 10⁻⁷ | 24 ± 2 |
| | 10⁻⁶ | 31 ± 3 |
| | 10⁻⁵ | 77 ± 11 |
| GLP-1 (7-37) | 10⁻⁸ | 128 ± 10 |
| Glucagon | 10⁻⁶ | 92 ± 9 |

| | | |
|---|---|---|
| *All cAMP levels are given as f-mole/5 ul of cell extract/15 min exposure to peptide | | |
| **Means ± S.E.M. (n = 4) | | |

## Claims

1. A compound which is:
(A) a peptide having the sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys; or
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly; or
(B) a peptide having at least 80% homology with one or other of the peptides of (A) with the proviso that the peptide is not GLP-1 7-36, GLP-1 7-37 or GLP-1 1-36; or
(C) a peptide which is:
(i) a pharmaceutically acceptable acid addition salt of the peptides of (A) or (B);
(ii) a pharmaceutically acceptable carboxylate salt of the peptides of (A) or (B);
(iii) a pharmaceutically acceptable lower alkyl ester of the (A) or (B) peptides; and/or
(iv) a pharmaceutically acceptable amide, alkyl amide, or dialkyl amide of (i), (ii), and/or (iii);
wherein the compound is substantially free of natural contaminants, and has an insulinotropic activity which exceeds the insulinotropic activity of GLP-1 (1-36) or GLP-1 (1-37).

2. A compound as claimed in claim 1 which is a peptide having the sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys.

3. A compound as claimed in claim 1 which is a peptide having the sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly.

4. A compound as defined in any one of claims 1 to 3 which is substantially free of natural contaminants, and has an insulinotropic activity at a concentration of at least 10⁻¹¹M.

5. A compound according to claim 4 which has an insulinotropic activity at a concentration of at least 10⁻¹⁰M.

6. A compound having the general formula:
(1)
H₂N--X--CO-R¹
wherein
R¹ represents OH, OM or -NR²R³;
M is a pharmaceutically acceptable cation or a lower branched or unbranched alkyl group;
R² and R³ are the same or different and each individually represents a hydrogen atom or a lower branched or unbranched alkyl group;
X is a peptide comprising the sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys; or
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
where NH₂ is the amine group of the amino terminus of X, and with the proviso that X is not GLP-1 7-36, GLP-1 7-37, GLP-1 1-36 or GLP-1 1-37.; and
CO is the carbonyl group of the carboxy terminus of X; or
(2) an acid addition salt thereof;
(3) the protected or partially protected derivatives thereof;
wherein the compound has an insulinotropic activity which exceeds the insulinotropic activity of GLP-1 (1-36) or GLP-1 (1-37).

7. An insulinotropic medicament comprising an effective amount of a compound according to any one of claims 1 to 6 in combination with a suitable physiologically acceptable carrier.

8. A compound according to any one of claims 1 to 6 for use in medicine.

## Patentansprüche

1. Zusammensetzung, welche entweder
(A) ein Peptid mit der Sequenz
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys
oder
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
oder(B) ein Peptid, das mindestens 80 % Homologie mit dem einen oder dem anderen der Peptide gemäß (A) aufweist, mit der Maßgabe, daß das Peptid nicht GLP-1 7-36; GLP-1 7-37 oder GLP-1 1-36 ist, oder
(C) ein Peptid ist, das
i) ein pharmazeutisch akzeptables Säureadditionssalz der Peptide gemäß (A) oder (B);
ii) ein pharmazeutisch akzeptables Carboxylatsalz der Peptide gemäß (A) oder (B);
iii) ein pharmazeutisch akzeptabler niedriger Alkylester der Peptide gemäß (A) oder (B); und/oder
iv) ein pharmazeutisch akzeptables Amid, Alkylamid, oder Dialkylamid von i), ii) und/oder iii) ist;
wobei die Verbindung im wesentlichen frei von natürlichen Verunreinigungen ist und eine insulinotropische Aktivität aufweist, die die insulinotropische Aktivität von GLP-1 (1-36) oder GLP-1 (1-37) übersteigt.

2. Verbindung nach Anspruch 1, welche ein Peptid mit der Sequenz
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys
ist.

3. Verbindung nach Anspruch 1, welche ein Peptid mit der Sequenz
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
ist.

4. Verbindung nach einem der Ansprüche 1 bis 3, welche im wesentlichen frei von Verunreinigungen ist und eine insulinotropische Aktivität bei einer Konzentration von wenigstens 10⁻¹¹ M aufweist.

5. Verbindung nach Anspruch 4, welche eine insulinotropische Aktivität bei einer Konzentration von wenigstens 10⁻¹⁰ aufweist.

6. Verbindung mit der allgemeinen Formel
(1)
H₂N--X--CO-R¹
in welcher R¹ OH, OM oder -NR²R³;
M ein pharmazeutisch akzeptables Kation oder eine niedrige verzweigte oder unverzweigte Alkylgruppe;
R² und R³ gleich oder verschieden und jeder ein Wasserstoffatom oder eine niedrige verzweigte oder unverzweigte Alkylgruppe darstellt;
X ein die Sequenz
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys
oder
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
aufweisendes Peptid, worin NH₂ die Aminogruppe des Amino-Terminus von X ist, mit der Maßgabe, daß X nicht GLP-1 7-36, GLP-1 7-37, GLP-1 1-36 oder GLP-1 1-37 ist, und
CO die Carbonylgruppe des Carboxy-Terminus von X ist; oder
(2) ein Säureadditionssalz davon ist;
(3) die geschützten oder teilweise geschützten Derivate davon sind;
wobei die Verbindung eine insulinotrope Aktivität aufweist, welche die insulinotrope Aktivität von GLP-1 (1-36) oder GLP-1 (1-37) übersteigt.

7. Ein insulinotropisches Medikament, welches eine wirksame Menge einer Verbindung gemäß einem der Ansprüche 1 bis 6 in Verbindung mit einem passenden physiologisch verträglichen Träger enthält.

8. Verbindung nach einem der Ansprüche 1 bis 6 für die Verwendung in der Medizin.

## Revendications

1. Composé qui est :
(A) un peptide ayant la séquence :
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys ; ou
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly ; ou
(B) un peptide ayant au moins 80% d'homologie avec l'un ou l'autre des peptides de (A) avec la condition que le peptide n'est pas GLP-1 7-36, GLP-1 7-37 ou GLP 1 1-36 ou
(C) un peptide qui est :
(i) un sel d'addition acide pharmaceutiquement acceptable des peptides de (A) ou (B);
(ii) un sel de carboxylate pharmaceutiquement acceptable des peptides de (A) ou (B) ;
(iii) un ester d'alkyle inférieur pharmaceutiquement acceptable des peptides de (A) ou (B) et/ou
(iv) un amide, un alkylamide ou un dialkylamide pharmaceutiquement acceptables de (i), (ii) et/ou (iii) ;
où le composé est substantiellement libre de contaminants naturels et a une activité insulinotrope qui dépasse 1' activité insulinotrope de GLP-1 (1-36) ou GLP-1 (1-37).

2. Composé selon la revendication 1 qui est un peptide ayant la séquence :
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys.

3. Composé selon la revendication 1, qui est un peptide ayant la séquence :
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly.

4. Composé comme défini dans l'une quelconque des revendications 1 à 3, qui est substantiellement libre de contaminants naturels, et a une activité insulinotrope à une concentration d'au moins 10⁻¹¹ M.

5. Composé selon la revendication 4, qui a une activité insulinotrope à une concentration d'au moins 10⁻¹⁰ M.

6. Composé ayant la formule générale :
(1)
H₂N- -X- -CO-R
¹ dans laquelle
R¹ représente OH, OM ou -NR²R³;
M est un cation pharmaceutiquement acceptable ou un groupement alkyle inférieur ramifié ou non ramifié ;
R² et R³ sont identiques ou différents et chacun d'eux représente individuellement un atome d'hydrogène ou un groupement alkyle inférieur ramifié ou non ramifié ;
X est un peptide comprenant la séquence : His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys ; ou
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly
où NH₂ est le groupement amine de l'amino terminal de X, et avec la condition que X n'est pas GLP-1 7-36, GLP-1 7-37, GLP-1 1-36 ou GLP-1 1-37 ; et CO est le groupement carbonyle du carboxy terminal de X ; ou
(2) un sel d'addition acide de celui-ci ;
(3) les dérivés protégés ou partiellement protégés de celui-ci ;
dans lequel le composé a une activité insulinotrope qui dépasse l'activité insulinotrope de GLP-1 (1-36) ou GLP-1 (1-37).

7. Médicament insulinotrope comprenant une quantité efficace d'un composé selon l'une quelconque des revendications 1 à 6 en combinaison avec un support approprié physiologiquement acceptable.

8. Composé selon l'une quelconque des revendications 1 à 6 pour l'utilisation en médecine.
